# EUROPÄISCHE PATENTANMELDUNG

(11) **EP 4 640 229 A1**
(43) Veröffentlichungstag der Anmeldung: **29.10.2025**
(21) Anmeldenummer: 24172710.6
(22) Anmeldetag: 26.04.2024
(51) Int. Cl.: A61K 36/537, A61K 35/644, A61P 31/04

(54) **ETHANOLISCHER AUSZUG GEGEN BORRELIA SPP AUSGELÖSTE ERKRANKUNGEN LYME-BORRELIOSE UND RÜCKFALLFIEBER**

(71) Anmelder: Patentpool Target GmbH, 80331 München (DE)
(72) Erfinder: Burda, Renate, 82054 Sauerlach (DE); Mörth-Kretschmer, Jenny, 21244 Buchholz in der Nordheide (DE); Sand, Elisabeth, 91567 Rauenzell/Herrieden (DE); Weickmann, Dirk, 91567 Rauenzell/Herrieden (DE)
(74) Vertreter: Reich, Jochen

(57) **Zusammenfassung**

Erfindungsgemäß wird ein ethanolischer Auszug aufweisend Salbei-Extrakte vorgeschlagen sowie ein Verfahren bzgl. dessen Herstellung.

## Beschreibung

Erfindungsgemäß wird ein ethanolischer Auszug aufweisend Salbei-Extrakte vorgeschlagen sowie ein Verfahren bzgl. dessen Herstellung.

In den 1980er-Jahren wurde FSME und FME beschrieben, wodurch durch Zecken übertragene Erkrankungen erstmals in den Fokus der Bevölkerung rückten. Die Frühsommer-Meningoenzephalitis ist eine durch das FSME-Virus ausgelöste Erkrankung, die mit ähnlichen Symptomen wie bei der Borreliose auftritt, also Fieber und partiell einer Meningoenzephalitis, der Entzündung von Gehirn und Hirnhäuten [1].

### Stand der Technik:

[1] Sebastian Wendt et al (2019): Durch Zecken übertragbare Erkrankungen: Von der Lyme-Borreliose über das Q-Fieber bis zur FSME. CME Band 16 (5): 53-71
[2] Hartmut Krauss et al (2004): Borreliosen. In: Hartmut Krauss, Albert Weber, Burkhard Enders (Hrsg.): Zoonosen. Von Tier zu Mensch übertragbare Infektionskrankheiten. 3. Auflage. Deutscher Ärzteverlag. Köln.
[3] Fukai Bao et al (2021/2022): Globale Seroprävalenz und soziodemografische Merkmale von Borrelia burdorferi senso lato in menschlichen Populationen: eine systematische Überprüfung und Metaanalyse. BMJ Global Health Band 7 Ausgabe 6.
[4] Dirk Weickmann, Toximed GmbH (2006): Pharmazeutischer Wirkstoff zur Behandlung von Hepatitis, Borreliose und Multipler Sklerose. European Patent Office EP 1754486A3
[5] Boiss. & Heldr. von Benth. (1848): Salvia pisidica. APde Candolle, Prodr. 12: 269.
[6] Gulcan Özkan, Sevil Albayrak (2010): Study on chemical composition and biological activities of essential oil and extract from Salvia pisidica. LWT - Food Science and Technology Volume 43, Issue 1, Pages 186-190.
[7] Renate Burda, Jenny Mörth-Kretschmer, Elisabeth Sand, Dirk Weickmann, Patentpool Target GmbH (2022): Kit of Parts zur Therapie der durch Borrelia spp. ausgelösten Borreliose. European Patent Office EP 4 233 547 A1
[8] Akgul H, Korkmaz N, Dayangac A, Sevindik M (2020): Antioxidant Potential of Endemic Salvia absconditiflora. Turkish Journal of Agriculture - Food Science and Technology, 8(10), 2222-2224
[9] Janicsak G, Zupko I, Hohmann J (2011): Bioactivity-guided Study of Antiproliferative Activities of Salvia Extracts. Natural Product Communications, Vol. 6, No. 5, 575-579
[10] Özcelik B, Erdogan Orhan I, Kan Y (2011): Determination of Antiviral Activity and Cytotoxicity of Selected Sage (Salvia L.) Species. FABAD J. Pharm. Sci., 36, 155-160, 2011
[11] Su CY, Ming QL, Rahman K, Han T, Qin LP. (2015): Salvia miltiorrhiza: Traditional medicinal uses, chemistry, and pharmacology. Chin J Nat Med. 2015 Mar; 13(3): 163-82
[12] Lin TH, Hsieh CL (2010): Pharmacological effects of Salvia miltiorrhiza (Danshen) on cerebral infarction. Chin Med 5, 22 (2010)

Borreliose wird von Bakterien und nicht wie FSME durch Viren ausgelöst. Die Übertragungsgefahr bestand "damals" nur bei jedem 10.000 oder 100.000 Zeckenbiss/-stich. Heutzutage kann man davon ausgehen, dass bei jedem Zeckenstich Borrelien übertragen werden. Die Gefährlichkeit der Borrelien und der Zusammenhang mit rheumatischen Erkrankungen wurde lange nicht erkannt, auch weil die Zeit zwischen einem Zeckenstich und dem Ausbruch der Krankheit bis zu 40 Jahre dauern kann. Meist vergehen Monate oder Jahre nach einem Zeckenbiss bis zum Ausbruch der Krankheit. Vermutlich wegen des allgemein schwächeren Immunsystems der "modernen" Bevölkerung und der allgemeinen Belastung der gesamten Umwelt mit Schadstoffen kommt es heute oft viel schneller zum Ausbruch der Krankheit mit zudem schwerwiegenderen Verläufen. Wir sind der Überzeugung, dass die meisten vor allem früher bei der ländlichen Bevölkerung aufgetretenen und auch heute noch auftretenden rheumatischen Beschwerden tatsächlich auf Borrelien-Infektionen zurückzuführen sind [2]. Neben der Lyme-Borreliose, welche durch die Borrelia-Arten:
- Borrelia burgdorferi
- Borrelia afzelii
- Borrelia garinii
- Borrelia bavariensis
- Borrelia mayonii
- Borrelia spielmanii
- evtl. Borrelia texanensis
ausgelöst wird gibt es noch eine weitere Erkrankung, die durch Borrelien ausgelöst und durch Zecken übertragen werden kann, das Rückfallfieber. Die Erreger wurden erstmals 1868 von dem deutschen Arzt und Bakteriologen Otto Obermeier (1843 - 1873) im Blut von Patienten nachgewiesen. Es handelte sich um die Art Borrelia recurrentis. Mittlerweile weiß man, daß auch Borrelia duttoni, B. crocidurae, B. hispanica, B. hermsii, B. venezuelensis, B. turicatae, B. mazzottii, B. persica und B. parkeri Rückfallfieber auslösen können.

Borreliose äußert sich anfangs mit unspezifischen Allgemeinsymptomen wie Fieber, Kopfschmerzen oder Magen-Darm-Beschwerden. Die Hautrötung (Erythema migrans oder Wanderröte) ist ein eindeutiges Symptom für eine Borrelieninfektion, die aber nicht bei allen Borreliose-Infektionen auftritt. Ein Fehlen der Wanderröte nach einem Zeckenbiss bedeutet nicht automatisch, dass keine Borreliose-Infektion erfolgt ist.

Symptome wie Grippe, Fieber und Kopfschmerzen erschweren das Erkennen der Krankheit. Es kann zu einem Befall der Organe, der Gelenke und Muskeln sowie des zentralen und peripheren Nervensystems kommen. Das Immunsystem ist in diesem Stadium oft nicht mehr in der Lage, die Infektionen zu bewältigen. Borrelien halten sich nur kurz im Blut auf und setzen sich im Bindegewebe und in Zellwänden fest, sie leben sozusagen versteckt. Hier sind sie schwer zu erkennen und deshalb ist eine länger andauernde Behandlung nötig.

Beim Rückfallfieber tritt mehrmals hohes Fieber welches durch Zytokine vermittelt wird auf. Zudem können die Erreger Schädigungen des Gefäßendothels auslösen, was zu einer zunehmenden Blutungsneigung und zu Nekrosen der befallenen Organe führt. Wenn der Erreger nicht vollständig eliminiert wird kommt es letztlich zu Sepsis und Multiorganversagen, was zum Tod des Patienten, der Patientin führt.

Weitere Symptome wie Gelenk-, Kopf-, Brust- und Gliederschmerzen aber auch Übelkeit und Atemnot können denen von Lyme-Borreliose stark ähneln.

### Stand der Technik bei der Behandlung:

Wenn die Infektion noch nicht weit fortgeschritten ist kann mit Antibiotika behandelt werden. Am effektivsten zeigt sich hier die Behandlung mit reinem Penicillin (C16H18N2O4S). Ob andere Antibiotika einen dauerhaften und nachhaltigen Effekt haben ist aktuell nicht bestätigt und im Moment noch anzuzweifeln. Die Behandlung mit Penicillin kann jedoch, wenn zufällig der richtige Zeitpunkt (bevor sich die Bakterien im Gewebe "verstecken") erkannt wird, hilfreich sein; jedoch diesen Zeitpunkt zu erkennen ist annähernd unmöglich. Ab dem Zeitpunkt der Übertragung des Erregers kann es nach einer unterschiedlichen Inkubationszeit zu einer Lokalinfektion der Haut kommen, die mit einer Hautrötung an der Stichstelle sichtbar wird. Die Inkubationszeit kann in manchen Fällen mehrere Jahre dauern, weshalb die damit verbundenen Gelenkschmerzen lange Zeit für rheumatische Beschwerden gehalten wurden und erfolglos mit Cortison-Salbe behandelt wurden. Wir sind der Überzeugung, dass die meisten vor allem früher bei der ländlichen Bevölkerung aufgetretenen und auch heute noch auftretenden rheumatischen Beschwerden auf Borrelien zurückzuführen sind.

### Probleme bei Diagnose und Therapie:

Entgegen der herkömmlichen Auffassung können sich Borellien sehr schnell ausbreiten. Einen absolut vorhersagbaren Verlauf, wie die klassische Einteilung in drei Stadien nahelegt, gibt es für die Borreliose nicht, der Ablauf entwickelt sich nicht gesetzmäßig. Passt das Beschwerdebild des Betroffenen nicht in die schulmedizinisch klassische Einteilung der Borreliose, so wird Borreliose oft ausgeschlossen und die Symptome werden falsch behandelt. Eine Borreliose-Infektion hält sich an kein Schema! Wie sich eine Borreliose entwickelt, lässt sich nicht vorhersagen. Die Symptomatik kann sich schnell und schwerwiegend entwickeln oder es kommt zu einer schleichenden und langsamen Manifestation.

Wenn Borreliose nicht rechtzeitig behandelt wird, kann es zu einer chronischen Infektion kommen. Das heißt, die Krankheit kehrt immer wieder oder verschlechtert sich zunehmend. Selbst jahrelange symptomfreie Latenzzeiten mit anschließender Reaktivierung sind möglich. Die Erreger lösen verschiedene Krankheitsbilder aus. Meist sich die Gelenke betroffen, es kann auch zu Herzproblemen und Gefäßentzündugen führen. Auch Mischformen der Erkrankungen sind möglich. Viele Borreliose-Patienten klagen über Erschöpfungszustände und chronische Müdigkeit, die durch ausreichend Schlaf nicht ausgeglichen werden kann. Ein für Menschen geeigneter Borreliose-Impfstoff ist in nächster Zukunft nicht zu erwarten. In den USA wurde ein entsprechender Impfstoff 2002 wegen angeblicher Impfkomplikationen wieder vom Markt genommen. Dieser Impfstoff war allerdings nur für den amerikanischen Erregerstamm geeignet und somit in Europa nicht anwendbar. Einzig echt wirksames Antibiotikum ist rein biologisches Penicillin. Dieses muss gemäß unseren Erfahrungen spätestens sechs Wochen nach der Infektion appliziert werden. Dies geschieht nur in den allerwenigsten Fällen.

Beim Rückfallfieber sind die Verläufe meist akut. Probleme gab es hier mit Penicillin, da es bei dessen Anwendung regelmäßig zu Rückfällen kommen kann.

Eine Gefahr die grundsätzlich bei der Behandlung von Borrelien besteht ist die sogenannte Jarisch-Herxheimer-Reaktion. Diese kann entstehen, wenn zu viele Borrelien auf einmal mittels Antibiotika abgetötet werden und durch die dabei freigesetzten bakteriellen Endotoxine ein Kreislaufschock bei den Behandelten indiziert wird, welcher mitunter tödlich enden kann.

### Borreliose aktuell:

Neuste Studien zeigen, dass heutzutage rund jeder 7. Mensch weltweit bereits mindestens 1x mit Lyme-Borreliose infiziert worden ist. Nach einer im BMJ Global Health publizierten Studie, weist Mitteleuropa mit 20% die höchste Infektionsrate auf [3]. Beim Rückfallfieber gibt es keine verlässlichen Zahlenquellen. Auszugehen ist allerdings davon, daß etwa jeder 15. - 20. Mensch weltweit zumindest schon einmal infiziert worden ist.

Somit ist es die Aufgabe dieser Erfindung eine wirksame Therapie gegen Borrelia-Arten zu entwickeln, die Lyme Borreliose und/oder Rückfallfieber auslösen können. Mit der Patentschrift WO2007060252A3 haben wir bereits seit 2006 ein Therapeutikum gegen Borreliose welches auch im Rahmen zulässiger ärztlicher Heilversuche positiv bewertet wurde. Hier fanden und entwickelten wir Wirkstoffe aus Gesamtgiftcocktails verschiedener Myriapoda [4]. Nachteil dieser Behandlungsmöglichkeit ist die verhältnismäßig schlechte Verfügbarkeit der Wirkstoffe. Mit der Patentschrift EP 4 233 547 A1 [7] haben wir 3 Salbei-Arten vorgestellt, welche Borrelia burgdorferi und/oder Borrelia afzelli abtöten können. Dies ist effektiv, genügt aber nicht vollständig einer Therapie gegen die von Borrelien ausgelösten Krankheiten Lyme-Borreliose und Rückfallfieber. Aus der Literatur ist bekannt, daß verschiedene Salbei-Arten möglicherweise gegen Tumorerkrankungen eingesetzt werden können [8, 9], ebenso geht man davon aus, daß Viren mittels der ätherischen Öle verschiedener Salbei-Species bekämpft werden können [10]. Eine Anwendung gegen verschiedene humanpathogene Bakterien ist nicht bekannt, speziell nicht gegen Borreliose. Hierzu gibt es nur die aus der TCM stammende Methode der Rotwurzelsalbei (Salvia miltiorrhiza) könne gegen Lyme-Disease eingesetzt werden [11].

In der vorliegenden Erfindung beschreiben wir eine effektive Behandlungsmöglichkeit der Lyme-Borreliose und des Rückfallfiebers bei bester Verfügbarkeit der Wirkstoffe und bei optimaler Verträglichkeit der Therapie für den Patienten/die Patientin.

### Erfindungsgemäße Zusammensetzung und Herstellung:

Im Folgenden werden Aspekte der vorliegenden Erfindung beschrieben.

Wir fanden nun überraschenderweise in mehreren Salbei-Arten [5]. Substanzen die Borrelia spp. und Yersinia spp. in vitro und in vivo abtöten können. Bislang geht man davon aus, dass ein Methanol-Extrakt, bzw. das Öl von verschiedenen Salvia spp. antimikrobielle Effekte gegen gram (+) Bakterien aufweist. Gegen gram (-) Bakterien ist kaum eine Wirkung nachweisbar. Unsere bisherigen Versuche bestätigen diese aktuelle wissenschaftliche Auffassung.

Wir fanden nun heraus, daß ein Ethanol-Extrakt der frischen Blätter der von uns in Tabelle A aufgelisteten Salvia spp. der in Honig eingearbeitet wird gram (-) Bakterien, speziell Borrelia spp. und/oder Yersinia spp. abtöten kann [6].

Somit eignet sich ein in Honig eingearbeiteter ethanolischer Extrakt der von uns untersuchten Salvia spp. zur Therapie von Lyme-disease und von Rückfallfieber.

Weltweit sind etwa 900 Arten Salbei bekannt, vermutlich gibt es weit über 1.000 Species. Viele haben pharmazeutisch-medizinisch interessante Substanzen. Nur wenige Arten wurden bislang untersucht. Wir untersuchten Extrakte der folgenden Salbeiarten hinsichtlich ihrer borrelienabtötenden Wirkung:
- Salvia mellifera
- Salvia absconditiflora
- Salvia caespitosa
- Salvia tomentosa
- Salvia taraxacifolia
- Salvia nevadensis
- Salvia fruticosa
- Salvia jurisicii
- Salvia recognita
- Salvia cinerea
- Salvia pisidica
- Salvia miltiorrhiza
- Salvia lavandulifolia
- Salvia officinalis Krk
- Salvia officinalis Dalmatien
- Salvia azurea

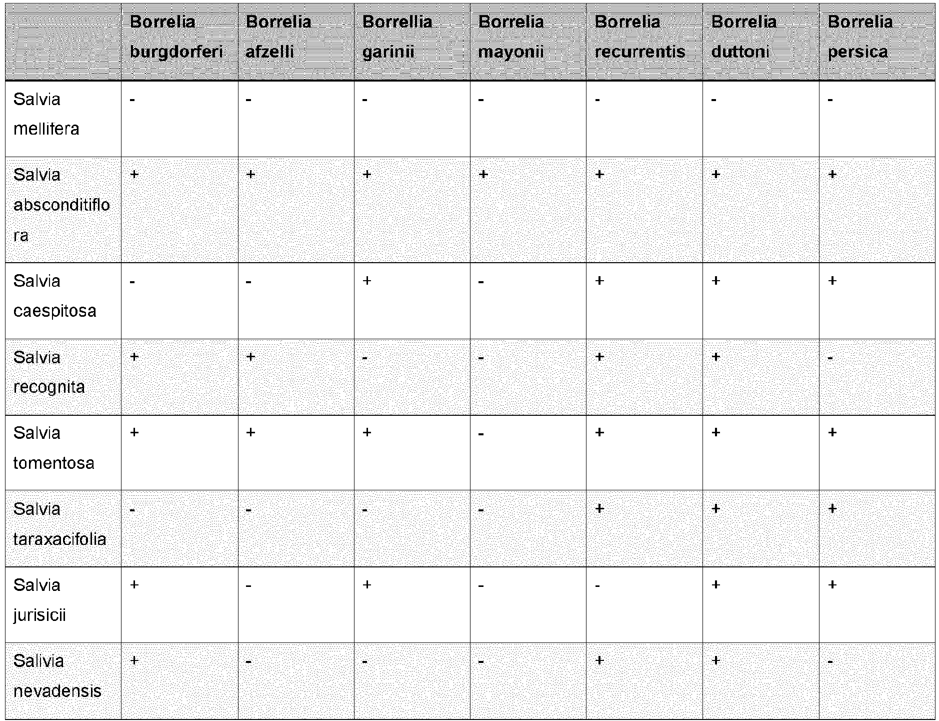

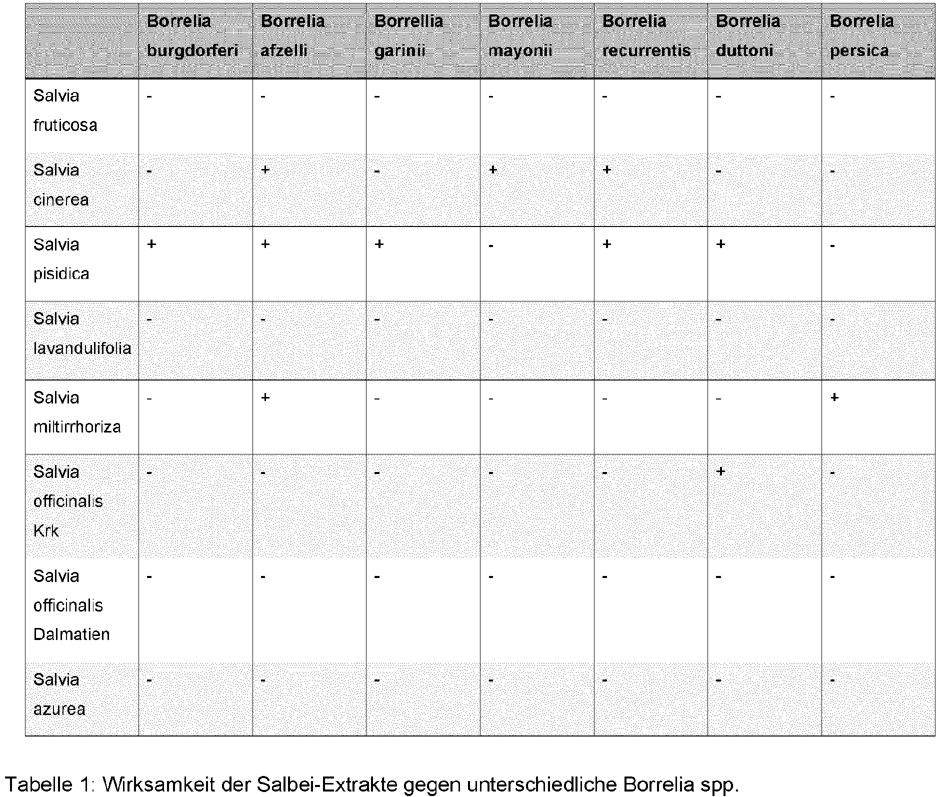

Nur Extrakte von Salvia absconditiflora, Salvia caespitosa, Salvia recognita, Salvia tomentosa, Salvia jurisicii und Salvia pisidica zeigten einen nachweislichen erfindungsgemässen Effekt gegen Borrelien. Gegen einzelne Borrelia-Arten waren noch Salvia cinerea, Salvia nevadensis, Salvia taraxacifolia und Salvia miltirrhoriza aktiv.

Erfindungsgemäß macht eine Mischung verschiedener Extrakte Sinn und ist nach dem jeweiligen Krankheitsbild zu kombinieren.

Folgende Salvia spp. sind alleine oder in Kombination als Extrakt für die erfindungsgemäße Anwendung bevorzugt, bzw. besonders bevorzugt:

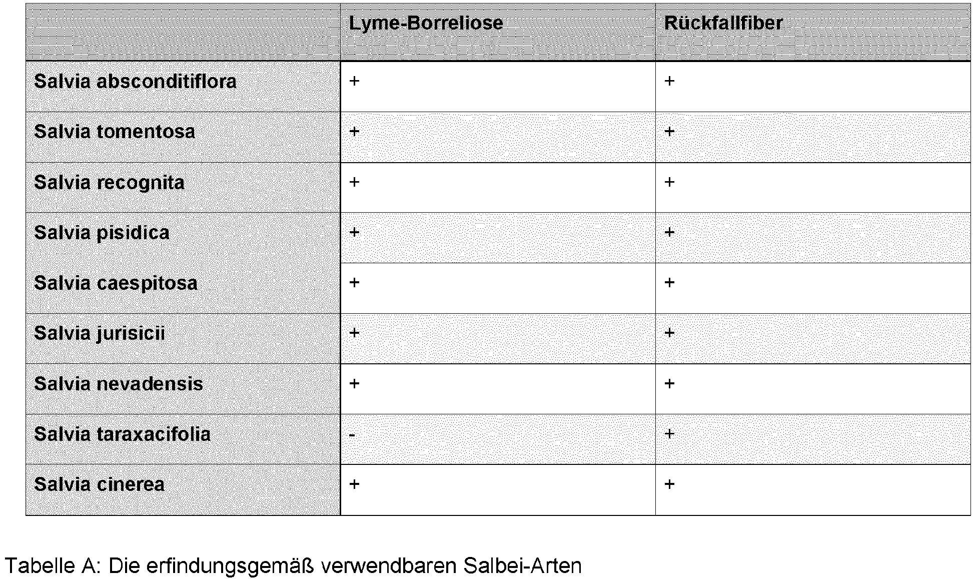

Die erfindungsgemäße Zusammensetzung wird auf die folgende Weise hergestellt:
a) Es wird eine bestimmte Menge Blätter, bevorzugt frische Blätter der Salvia spp. in eine bestimmte Menge 96% Ethanol, bzw. bevorzugt 100% Ethanol eingebracht.
b) Der Extrakt aus a) wird mindestens 24 Stunden bei Temperaturen von bevorzugt 25 Grad Celsius stehen gelassen. Anschließend werden die Blätter abfiltriert.
c) Dieser Wirkextrakt aus b) wird in 150 mL verflüssigten Bioblütenhonig, besonders bevorzugt Bio-Rosmarinhonig unter ständigem Rühren eingearbeitet.

### Kultur der Salvia spp. zur optimalen Wirkstoffsynthese:

Damit Pflanzen, vor allem die für die erfindungsgemäße Verwendung benötigten Arten Salvia absconditiflora, Salvia tomentosa, Salvia recognita, Salvia pisidica, Salvia caespitosa relevante Mengen ihrer Wirkstoffe produzieren können, ist es wichtig die richtigen Kultursubstrate/Böden für die Pflanzen zu haben.

Wir fanden heraus, dass für die erfindungsgemässen Salvia spp. nicht nur ein durchlässiger Boden vonnöten ist, sondern das die Arten zur Wirkstoff-Synthese einen kalkhaltigen Boden benötigen. Bevorzugt verwenden wir Kalkerde von flora montana mit ca. 20%-35% verwitternden Kalkstein.

Mehr als 35 verschiedene Komponenten lassen sich im Ehtanol-Extrakt der von uns verwendeten Salvia spp. nachweisen. Gallensäure-Äquivalente, Rutin-Äquivalente und Catechin-Äquivalente können durch die richtige Substratverwendung gesteigert werden. Zudem konnten wir Peptidtoxinähnliche Substanzen, welche noch nicht chemisch beschrieben sind nachweisen. Eine Kultur in Pflanzegefäßen ist effizienter als eine solche direkt im Boden.

Für die erfindungsgemäße Anwendung ist es möglich, die frischen Blätter der Pflanzen ohne Stängel zu verwenden. Wirkungen konnten wir gemäß einem Aspekt der vorliegenden Erfindung beobachten bei folgenden Konzentrationen:

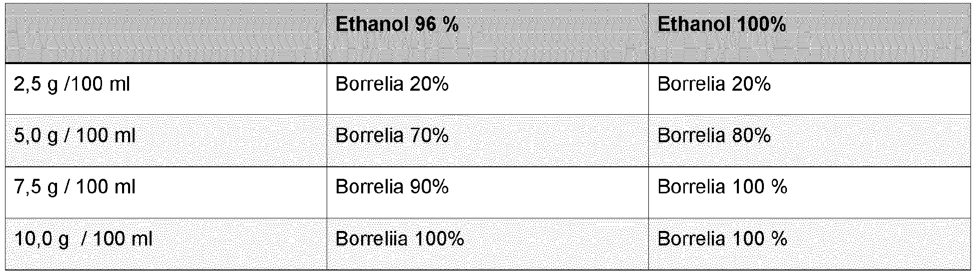

Somit sind die höheren Konzentrationen und die Auszüge in 100% Ethanol am effektivsten.

### Darreichungsformen:

Es zeigt sich, dass die Extrakte oral eingenommen werden können. Die Wirkstoffaufnahme geschieht über den Verdauungstrakt. Der ethanolische Auszug ist besonders gut in Honig lösbar, bevorzugt in Rosmarinhonig, so ist neben der direkten, puren Einnahme des ethanolischen Extraktes auch eine solche gelöst in Honig, besonders bevorzugt Bio-Rosmarinhonig sinnvoll.

Im Rahmen der vorliegenden Erfindung gelten junge Blätter als Blätter die maximal 6 Tage, bevorzugt maximal 4 Tage alt sind. Wobei das Alter ab Beginn der Öffnung der Blattknospe gezählt wird. Dies ist vorteilhaft, da in den jungen Blättern die höchsten Anreicherungen der pharmazeutischen Wirkstoffe vorhanden sind. Die jungen Blätter schützen sich durch diese Eigenschaft in jungem Studium gegen Fressfeinde und Krankheitsereger.

Im Rahmen der vorliegenden Erfindung gelten frische Blätter als Blätter die maximal seit 24 bis höchstens 72 Stunden geerntet sind d.h. typischerweise geschnitten wurden.

Dies ist vorteilhaft, da nach der Ernte das Blatt chemisch und physisch einer Änderung unterliegt und die bevorzugten Eigenschaften dann abnehmen.

Mindestens 24 Stunden stehen lassen bedeutet gemäß einem Aspekt der vorliegenden Erfindung dass dieser Extrakt mindestens 24 Stunden unbehandelt bleibt. Dies ist vorteilhaft, da in 24 Stunden die höchste Anreicherung des Extrakts mit den gewünschten Inhaltsstoffen erfolgt. Hierbei ist es vorteilhaft den Extrakt ca. alle 2 Stunden zu schränken. Schränken bedeutet Schwenken, was bevorzugt leicht, derart, dass die Oberfläche physisch verändert wird, durchgeführt wird. Somit wird eine homogene Mischung geschaffen die wiederum neue Wirkstoffextraktionen begünstigt. Bevorzugt erfolgt dies gegen den Uhrzeigersinn, was den Vorteil schafft, dass die linksdrehenden Wirkstoffe besser extrahiert werden.

Etwa 25 Grad Celsius bedeutet gemäß einem Aspekt der vorliegenden Erfindung eine bevorzugte Temperatur von 24 bis 26 Grad Celsius. Dies ist vorteilhaft, da hier vorteilhafteste Wirkstoffextraktion erfolgt. Dies wurde empirisch nachgewiesen.

Bei verflüssigtem Bioblütenhonig kann es sich um 150 ml handeln, um eine Standardisierung zu erreichen. Dies ist vorteilhaft, da im Heilversuch somit eine Monatsdosis des Extrakts geschaffen werden kann. Dies ist vorteilhaft da sich der Extrakt für ca. einen Monat ohne relevante Veränderung hält.

Blütenhonig und/ oder Rosmarinhonig werden gemäß einem Aspekt der vorliegenden Erfindung, aber optional, in Bio-Qualität bereitgestellt. Bio-Qualität bedeutet, dass der Honig schadstoffarm und giftarm ist. Dies ist vorteilhaft, da das Extrakt bei geschädigten Menschen zum Einsatz kommt und somit eine weitere Belastung reduziert bzw. nahezu ausgeschlossen wird.

Zumindest 96% Ethanol bedeutet, dass Ethanol mit einem Volumenanteil von mindestens 96% bereitgestellt wird, also circa 96%, 97%, 98%, 99% oder 100% Volumenanteil.

Dies ist vorteilhaft, da ab 96% Ethanol das beste Lösungsverhalten entsteht.

"Bekämpfung der Borreliose" bedeutet, dass die Konzentration der Borrelien in einem Blutplasma bei höchstens 1% liegt. Die Symptome gehen also zurück oder verschwinden bevorzugt ganz. Die Borrelien, also die auslösenden Erreger der Borriolose und des Rückfallfiebers reduzieren sich bevorzugt auf Null. Somit sind auch entsprechende Toxine nicht nachweisbar.

Der Mengenanteile Salbei, Ethanol und Honig ist bevorzugt bei ca. ¾ Honig und ¼ Extrakt, wobei der Extrakt wiederum aus Ethanol und Salbei besteht oder aus Wasser und Salbei. Der Extrakt besteht bevorzugt aus maximal 10% Salbei und mindestens 90% Ethanol oder Wasser. Ethanol und Wasser können jeweils miteinander ausgetauscht oder kombiniert werden.

Der verwendete Salbei kann aus einer oder mehrerer Arten von Salbei gewählt werden. Es ist also eine Kombination mehrerer Arten möglich.

### Patientenbeispiele:

1. Frau 48 Jahre wurde mehrfach infiziert und bekam nach Gabe von Espicillin trotzdem eine in Schüben auftretende Lyme-Borreliose. Sodann nimmt die Frau einen in Honig gelösten alkoholischen (in 96% Ethanol) Extrakt von Salvia taraxacifolia und Salvia absconditiflora (zu gleichen Teilen). Einnahme tgl. 2cl immer abends. Sodann ist die Frau frei von Beschwerden und es sind keine Borrelien mehr nachweisbar. Seit dann nimmt die Frau gelegentlich noch vom Salbei-Extrakte in Honig.
2. Mann 36 Jahre beobachtete nach einem Zeckenbiss eine große kreisförmige Rötung um die Bissstelle und ging daraufhin zum Arzt. Dieser schickte ihn ins KH wo er eine Antibiotika-Infusion erhielt. Es treten Borreliose-Symptome auf. Teilweise kann der Mann nur mit Rollator laufen. Der Arzt bestätigt Lyme-Borreliose. Sodann nimmt der Mann einen wässrigen (verträgt keinen Alkohol) Extrakt von Salvia pisidica und Salvia tomentosa (hiervon waren die meisten Blätter vorhanden) der in Honig gelöst wurde. Der Mann nimmt tgl. abends 2cl.
   Ab Sommer meint der Mann, dass die Symptome besser werden aber noch kein durchgreifender Erfolg vorhanden ist. Daraufhin werden dem Extrakt noch in gleicher Menge Blätter von Salvia absconditiflora zugefügt. Ab dann klingen die Symptome ab und sodann ist der Mann bis heute beschwerdefrei. Seit dieser Zeit macht er 1 bis 2 Mal im Jahr eine Kur mit den Salbei-Extrakten über jeweils 20 Tage abends 2cl.
3. Frau 56 Jahre plagt sich seit Jahren mit MS. Nach längeren Gesprächen und Durchsicht aller Unterlagen stellt sich auch bei dieser Frau heraus, dass die Diagnose falsch war und allem Anschein nach eine Lyme-Borreliose vorliegt. Nach labortechnischer Abklärung dieser Vermutung und der Bestätigung des Vorliegens einer südlichen Borreliose beginnt die Frau mit der Einnahme eines alkoholischen (96%) Extraktes aus Salvia absconditiflora und Salvia decumbens (Salbei-Auswahl wegen Verfügbarkeit) in Honig. Die Frau nimmt tgl. abends 2cl zu sich. Sodann ist die Frau beschwerdefrei und es sind keine Borrelia spp. mehr nachweisbar.

## Patentansprüche

1. Ethanolischer Auszug von Blättern einer der Salvia spp. zur Verwendung in einem Verfahren zur Bekämpfung der Borreliose aufweisend folgende Bestandteile:
a) Blätter der Salvia spp. ausgewählt aus der Gruppe der Salbei-Arten bestehend aus Salvia absconditiflora, Salvia tomentosa, Salvia recognita, Salvia pisidica, Salvia caespitosa, Salvia jurisicii, Salvia nevadensis, Salvia taraxacifolia, Salvia cinerea und/ oder Kombinationen davon;
b) Zumindest 96% Ethanol; und
c) Bienenhonig.

2. Ethanolischer Auszug zur Verwendung gemäß Anspruch 1, wobei die Blätter jung sind.

3. Ethanolischer Auszug zur Verwendung gemäß Anspruch 1 oder 2, wobei es sich bei dem Ethanol um 100% Ethanol handelt.

4. Ethanolischer Auszug zur Verwendung gemäß einem der Ansprüche 1 bis 3, **dadurch gekennzeichnet, dass** der Bienenhonig die Qualität von Bioblütenhonig aufweist und/ oder Rosmarinhonig aufweist

5. Verfahren zur Herstellung einer therapeutischen Zusammensetzung zur Bekämpfung der Borreliose, aufweisend die Schritte:
a) ein Einbringen von Blättern der Salvia spp. ausgewählt aus der Gruppe der Salbei-Arten bestehend aus Salvia absconditiflora, Salvia tomentosa, Salvia recognita, Salvia pisidica, Salvia caespitosa, Salvia jurisicii, Salvia nevadensis, Salvia taraxacifolia, Salvia cinerea und Kombinationen davon in eine Menge von mindestens 96% Ethanol;
b) ein Extrakt aus a) wird mindestens 24 Stunden bei Temperaturen von etwa 25 Grad Celsius stehen gelassen;
c) ein Abfiltrieren der Blätter;
d) ein Einarbeiten des verbleibenden Extrakts aus c) unter ständigem Rühren in verflüssigten Blütenhonig.

6. Verfahren nach Anspruch 5, wobei der Blütenhonig Rosmarinhonig aufweist.

7. Verfahren nach mindestens einem der Ansprüche 5 oder 6, wobei der Blütenhonig und/ oder Rosmarinhonig Bio-Qualität aufweist.
